# EUROPEAN PATENT SPECIFICATION

(11) **EP 0 843 680 B1**
(45) Date of publication and mention of the grant of the patent: **12.09.2001**
(21) Application number: 96927643.5
(22) Date of filing: 30.07.1996
(51) Int. Cl.: C07D 503/00

(54) **PROCESS FOR THE PREPARATION OF POTASSIUM CLAVULANATE**
VERFAHREN ZUR HERSTELLUNG VON KALIUMCLAVULANAT
PROCEDE POUR LA PREPARATION DE CLAVULANATE DE POTASSIUM

(30) Priority: 02.08.1995 GB 9515809
(43) Date of publication of application: 27.05.1998
(60) Divisional application: 00127571.8
(73) Proprietor: SMITHKLINE BEECHAM PLC, Brentford, Middlesex TW8 9EP (GB)
(72) Inventor: COOK, Michael Allen, SmithKline Beecham Pharma., Worthing, West Sussex BN14 8QH (GB)
(74) Representative: Walker, Ralph Francis, Dr.
(86) International application number: EP9603375
(87) International publication number: WO9705142

(56) References cited:
- EP-A- 0 026 044
- EP-A- 0 277 008
- EP-A- 0 312 813
- WO-A-95/23870
- WO-A-96/28452
- AT-B- 400 846
- GB-A- 2 003 863
- GB-A- 2 287 025

## Description

This invention relates to a novel process for the preparation of salts of clavulanic acid.

Clavulanic acid (I): 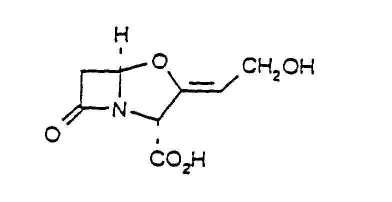 is a β-lactamase inhibitor which is used commercially as a component of pharmaceutical formulations, usually in the form of its salts. Clavulanic acid is produced commercially by culture of the microorganism *Streptomyces clavuligerus,* for example as described in GB-A-1508977.

Clavulanic acid or its salts may be extracted from the culture medium in various ways but one way this is achieved is that the cells of the *S. clavuligerus* are first removed from the culture medium by such methods as filtration or centrifugation before such extraction procedures are commenced.

Clavulanic acid or its salts may be extracted from clarified culture medium by a variety of methods. Solvent extraction from cold clarified culture medium adjusted to acid pH values and methods which utilize the anionic nature of clavulanic acid at neutral pH such as the use of anion exchange resins have been found to be particularly useful. A further particularly useful method is to form an ester of clavulanic acid, purify the ester and regenerate the acid or its salt therefrom.

The extraction processes for obtaining clavulanic acid or its salts may notionally be divided into a primary isolation process followed by a further purification process.

Suitable primary isolation processes include solvent extraction of the free clavulanic acid. In the solvent extraction process the clavulanic acid is extracted into an organic solvent from cold clarified culture medium, which may be whole broth, adjusted to an acid pH value.

In one solvent extraction process for clavulanic free acid the clarified medium is chilled and the pH lowered into the region of pH 1-2 by the addition of acid while mixing with a substantially water-imiscible organic solvent. Suitable acids used to lower the pH include hydrochloric, sulphuric, nitric, phosphoric or the like mineral acids. Suitable organic solvents include n-butanol, ethyl acetate, n-butyl acetate and methyl isobutyl ketone, and other similar solvents. Methyl isobutyl ketone is a particularly suitable solvent for use in the extraction of the acidified culture filtrate. After separation of the phases clavulanic acid is found in solution in the organic phase.

The clavulanic acid may be back extracted from the organic phase into a new aqueous phase by making use of the greater water solubility of, for example, the alkali metal or alkaline earth metal salts of clavulanic acid in water than in organic solvents. Thus the clavulanic acid may be back extracted from the organic solvent into an aqueous solution or suspension of an alkali metal or alkaline earth metal base, such as sodium hydrogen carbonate, potassium hydrogen phosphate buffer or calcium carbonate, or water, while maintaining the pH at approximately neutrality, for example pH 7. This aqueous extract, after separation of the phases, may be concentrated under reduced pressure. Freeze-drying may also be employed to provide a solid crude preparation of the salt of clavulanic acid. Such solid preparations are stable when stored as a dry solid at -20°C. A similar process is described in GB-A-1563103. This process may be modified in known ways by for example additional purification steps applied to the organic solvent phase to remove high molecular weight impurities from the impure clavulanic acid.

A further secondary purification process for clavulanic acid is that described in for example EP-A-0026044, in which a solution of impure clavulanic acid in an organic solvent is contacted with t-butylamine to form the t-butylamine salt of clavulanic acid, which is then isolated, thereby separating the clavulanic acid from impurities remaining in the organic solvent, and the salt is then converted back to clavulanic acid or into a derivative of clavulanic acid such as an alkali metal salt or an ester. Other known secondary purification processes for clavulanic acid involve the use of other organic amines such as diethylamine, tri-(lower alkyl) amines, dimethylaniline and NN'-diisopropylethylenediamine to form salts and/or other derivatives thereof with the clavulanic acid. These purification process have the inherent disadvantage that they can introduce traces of the amine, or leave residual traces of salts of clavulanic acid with the amine, in the final product.

Such back extraction processes present a problem when potassium clavulanate is prepared, as potassium clavulanate is particularly water-sensitive. In conventional back extraction processes potassium clavulanate can remain in contact with water for a long time, typically around an hour or more as the solution concentration of potassium clavulanate builds up under the relatively gentle mixing and separating conditions generally used, and this can lead to extensive hydrolytic degradation. Furthermore such a process involving the participation of clavulanate acid via certain amine salts as described above results in a relatively expensive process.

Other processes for preparing salts of clavulanic acid are disclosed in EP-A-312813 in which a lithium salt is first formed, AT-B-400846 in which clavulanic acid is reacted with an alkali metal salt, WO-A-95/21173 in which a salt precursor is reacted with clavulanic acid in turbulent conditions, and GB-A-1508977 (= BE-A-827926) in which alkali metal clavulanate salts are isolated from solution by freeze-drying. WO 96/28452 discloses a process in which potassium clavulanate is prepared by reaction of clavulanic acid with a potassium salt in an organic solvent medium.

The inventors have discovered an improved process for the preparation of salts of clavulanic acid in which degradation and cost is reduced.

Accordingly the present invention provides a process for the preparation of pharmaceutically acceptable quality potassium clavulanic acid by the direct precipitation of clavulanic acid as the potassium salt which has not been pre-purified by the formation of an intermediate amine salt, according to claim 1.

Pharmaceutically acceptable quality potassium clavulanate encompasses material which has a purity of at least 80% as the pure free acid more preferably a purity of at least 82% as the pure free acid and most preferably is purity of at least 83 % as the pure free acid.

The above process may be effected by extraction into an organic solvent of the free clavulanic acid resulting from a fermentation process as described in the references above and which may be optionally pre-treated according to the procedures mentioned in the documents above or preferably using techniques hereinafter described. Suitable organic solvents may be used as described in the aforementioned documents.

The extracted clavulanic acid may then be optionally concentrated using techniques conventional in the art or mentioned in documents cited herein or preferably hereinafter described.

Suitable potassium salts are salts with counter anions including basic anions, such as hydrogen carbonate, carbonate or hydrogen phosphate, and in particular anions of weak organic carboxylic acids, such as those of formula R-CO₂H where R is C₁₋₂₀ alkyl, for example C₁₋₈ alkyl. Suitable carboxylic acids include acetic, propionic and ethyl hexanoic, such as 2-ethyl hexanoic acid.

Suitable potassium salts including these ions are potassium hydrogen carbonate, potassium hydrogen phosphate, and particularly potassium 2-ethyl hexanoate.

The potassium salt is suitably a salt of an organic acid.

The thus formed potassium salt may then be conventionally isolated and dried or may be isolated and dried using the methods described herein.

Alternatively, the free acid of clavulanic acid (prepared as above) may be extracted into solvent as described above and optionally concentrated before back extraction of the clavulanic acid into an aqueous medium. The aqueous medium may then be optionally concentrated using conventional techniques or techniques described herein. A suitable potassium salt may suitably be added such as a potassium salt of an acid such as an organic acid. The resulting potassium clavulanate may be precipitated, isolated and dried, for example, by adding an appropriate precipitating solvent such as for example acetone or isopropanol.

Preferably a co-solvent may be added to the aqueous concentrate such as isopropanol and any resulting oil is removed by conventional means such as filtration through a celite bed. This is particularly advantageous in allowing the required purity of potassium clavulanate to be formed.

The product is then suitably precipitated by addition of further water miscible solvent such isopropanol optionally admixed with a water immiscible solvent such as ethyl acetate or alternatively by the step-wise addition of the water miscible solvent then the water immiscible solvent.

The techniques described in the examples are herein incorporated by reference.

It is especially desirable to use organic solvent extracts of clavulanic acid or aqueous extracts of clavulanic acid which are particularly high in titre for example the titre of clavulanic acid in aqueous solution is in the range of 100,000 to 200,000 micrograms per ml more preferably at about 145,000 micrograms per ml.

Preferably the clavulanic acid is obtained from a whole broth fermentation which is preferably filtered by ultra filtration preferably optionally pre-concentrated by reverse osmosis. The aqueous back extract is preferably at a concentration of 10 to 30% more preferably at a concentration of 15 to 25% and most preferably at about 20% w/v.

Suitable organic solvents include those described above, for example n-butanol, ethyl acetate, n-butyl acetate, and ketones of the general formula R¹ CO.R² where R¹ and R² are independently C₁₋₁₀ alkyl groups, in particular methyl isobutyl ketone. The solution of clavulanic acid may contain impurities, for example high molecular weight impurities such as may be present if the solution has been obtained by a primary isolation process as described above, but preferably has been subjected to a preliminary purification process to remove at least some of the impurities. Suitable preliminary purification processes include filtration, and treatment with absorbent carbon. The solution may also contain small quantities of dissolved or suspended water, but preferably if the solution has been obtained from a primary isolation process it may be subjected to a dewatering process, for example centrifuging to remove droplets of suspended water.

A suitable solution concentration for the solution of clavulanic acid or its labile derivative is around 500 to 20,000 µg/ml (0.0025M to 0.1M), for example around 1,000 - 5,000 µg/ml (i.e. 0.005M to 0.025M), typically around 3,000 ± 1,000 µg/ml (i.e. 0.015 M ± 0.005 M) expressed in terms of clavulanic acid content. Suitable labile derivatives of clavulanic acid include readily-cleaved esters, such as silyl esters. The term "clavulanic acid" as herein after used refers to both free clavulanic acid and such labile derivatives.

The clavulanic acid may be contacted in solution with the potassium salt by dissolving or suspending the salt in a solvent, and mixing the two solutions or the solution and suspension. The same organic solvent may be used for the clavulanic acid and the potassium salt. In the case of potassium 2-ethyl hexanoate as potassium salt such a solution in an organic solvent such as methyl isobutyl ketone may suitably be 0.5 to 5.0 M, e.g. 1.0 to 3.0 M, suitably 2.0 ± 0.5M in potassium 2-ethyl hexanoate.

Water may be provided in the contact region in a number of ways as discussed below, and one or more of these ways may be used as alternatives or together. For example the potassium salt may itself be dissolved or suspended in water or water containing dissolved organic solvent, and contacted as such with the solution of clavulanic acid. For example the solution of clavulanic acid may contain dissolved or suspended water, e.g. as mentioned above. For example the potassium salt may be dissolved or suspended in an organic solvent, e.g. the same solvent as the clavulanic acid is dissolved in, and the solvent may itself include dissolved or suspended water. For example methyl isobutyl ketone may be used as such an organic solvent for the clavulanic acid and the potassium salt, and may include 0.1 to 7.5% v:v of dissolved water, typically 1 to 3%, e.g. 2.0 ± 0.5%. For example water may be provided by adding water or an aqueous medium such as water containing dissolved organic solvent to the clavulanic acid solution and solution or suspension of the potassium salt in an organic solvent, as they are brought into contact in the contact region.

When dissolved water is present in the organic solvents used in the process of the invention, e.g. as described above, it may subsequently separate out as the aqueous phase by a "salting out" effect as it accumulates the dissolved clavulanic acid salt.

The working conditions, e.g. concentrations of reactants, relative proportions of solutions used, flow rates, contact times etc., of the process are selected such that *inter alia* as much as possible of the clavulanic acid is extracted from the solution in the organic solvent into the aqueous phase as the solution of potasium clavulanate, and such that a concentrated solution of potassium clavulanate in the aqueous phase is formed. In the case of potassium clavulanate, in a preferred embodiment working conditions are selected so as to produce a concentration of potassium clavulanate in the aqueous phase of ca. 10 to 40 weight % (ca. 0.4 to 1.7 M), for example 20 to 30 weight % (ca. 0.8 to 1.2 M). A solution concentration of potassium clavulanate of this concentration is found to expediate the further processing step, with an optimised yield and improved purity.

In the process, monitoring of the concentration of the potassium clavulanate in the separated aqueous phase, for example by density, optically etc. is a suitable way of determining and controlling the other working conditions.

The clavulanic acid and the potassium salt should be introduced such that there is an initial stoichometric excess of the salt over the clavulanic acid. For example the potassium salt may be introduced in a 1 : 1.1 to 1 : 2 molar ratio clavulanic acid : potassium salt, typically 1 : 1.1 to 1 : 1.5, to ensure that there is theoretically sufficient potassium salt to combine with all of the clavulanic acid.

The amount of water present in the region in which the clavulanic acid and potassium salt are brought into contact should suitably be around the minimum necessary to achieve a desired aqueous phase concentration of the potassium clavulanate.

The process of this invention provides potassium clavulanate, free of the trace impurities introduced by known purification processes, such as the amines used in the purification process mentioned above. Although salts of clavulanic acid free of such impurities are known on a laboratory scale, the bulk production of such salts, in particular potassium clavulanate, for use in the preparation of pharmaceutical formulations is novel.

The product of the process of this invention may be used in a pharmaceutical formulation for the treatment of bacterial infections which comprises potassium clavulanate, the formulation being substantially free of organic amines such as t-butylamine, diethylamine, tri-(lower alkyl) amines, methylaniline or NN'-diisopropylethylene diamine (either as free amines or as derivatives or salts thereof).

Suitably the formulation contains less than 0.05%, for example less than 0.005%, preferably less than 0.0005%, desirably less than 0.00005% of organic amines by weight with respect to the weight of the potassium clavulanate present in the formulation.

The formulation may also comprise one or more antibiotic compounds, suitably β-lactam antibiotics such as penicillins and cephalosporins. Suitable antibiotics include the antibiotics with which potassium clavulanate is combined in known antibiotic formulations, for example amoxycillin (e.g. in the form of its trihydrate) and ticarcillin. The formulation may comprise ratios of potassium clavulanate : antibiotic within the known ranges in which such combinations are used, e.g. 12:1 to 1:1 by weight expressed as in terms of the parent clavulanic acid and antibiotic.

The formulation may also contain other known additives and excipients, e.g., fillers, binders, disintegrants, an effervescent couple, colourants, flavourings, desiccants etc., for example those listed for use with formulations containing potassium clavulanate in GB 2005538. The formulation may also contain materials such as cellulose derivatives, e.g. microcrystalline celluloses such as Avicel (Trade Mark) or Syloid (Trade Mark), silicon dioxide or sucrose together with potassium clavulanate. The formulation may for example comprise a blend of potassium clavulanate with a cellulose derivative, silicon dioxide or sucrose, for example in a 1:1 weight ratio.

The following examples illustrate the present invention:

### Example 1

A crude aqueous solution of clavulanic acid at a titre of about 20,000µg/ml was prepared by either of the following procedures:
(a) RVF filtration of the broth followed by ion exchange chromatography of fermentation broth.
(b) Ultrafiltration of the broth followed by RO concentration of fermentation broth.

The crude aqueous solution of clavulanic acid at a titre of about 20,000µg/ml, prepared by either a) or b) as above was extracted with MIBK at pH1.5, acidified with sulphuric acid. The rich solvent was chilled and dewatered prior to passing through activated granular carbon column. The purified solvent extract was then back extracted with water at pH5.1 using KEH in a solvent. The whole operation was continuous. The spend solvent was recycled back to the forward extraction and the aqueous extract was recycled until a concentration of 20-25 % w/v was reached. The aqueous potassium clavulanate solution was diluted with IPA, treated with powdered carbon then crystallised by the addition of IPA or acetone. A co-solvent may be used at this state.

### Example 2

A crude solution of clavulanic acid was prepared by ultrafiltration of clavulanic acid whole broth followed by RO concentration. An aqueous back extract at a concentration of 20.3% was prepared as in Example 1. IPA (100ml) was added to a 100ml of this solution. The resulting oil was removed by filtration through a celite bed. IPA (2,000ml) was slowly added to the filtrate whilst stirring. At the end of the IPA addition, ethyl acetate (300ml) was added and the resultant slurry was chilled and stirred for 1 hour prior to filtration and product drying.

| | |
|---|---|
| Product weight | = 21.0g |
| Crystallisation stage yield | = 86.3% |
| Product purity | = 83.2% as the pure free acid |
| Product moisture content | = 0.3% |

## Claims (Claims for the following Contracting State(s): BE, CH, LI, DE, DK, ES, FI, FR, GB, GR, IE, IT, LU, MC, NL, PT, SE)

1. A process for the preparation of pharmaceutically acceptable quality potassium clavulanate by the direct precipitation of clavulanic acid as the potassium salt which has not been pre-purified by the formation of an intermediate amine salt, by the reaction of clavulanic acid in solution with potassium ions in solution, in which the free acid of clavulanic acid resulting from a fermentation process is extracted into an organic solvent to thereby form a solution of clavulanic acid in the organic solvent, ***characterised* by**:
adding a potassium salt to the clavulanic acid in solution in the organic solvent,
then back extracting the so-formed potassium clavulanate into an aqueous medium from the organic solvent to thereby form a solution of potassium clavulanate in the aqueous medium as a separate phase to the organic solvent,
then causing the potassium clavulanate product to precipitate from the aqueous solution by the addition of an organic precipitating solvent to the aqueous solution of potassium clavulanate,
with the proviso that:
a process for the preparation of potassium clavulanate, wherein clavulanic acid in solution in a wholly or partly water-imiscible organic solvent is contacted in a contact region which is a region of high turbulence and/or shear stress, with a compound of potassium with a counter anion in solution or suspension, the counter anion being capable of exchange with clavulanate anion, in the presence of water, such that a solution of potassium clavulanate in an aqueous phase is formed, then the organic solvent and aqueous phases are physically separated during a separation step, followed by a further processing step in which the said salt of clavulanic acid is isolated from solution as a solid, is excluded.

2. A process according to claim 1 **characterised in that** the pharmaceutically acceptable quality potassium clavulanate has a purity of at least 80% as the pure free acid.

3. A process according to claim 1 or 2 **characterised in that** the potassium salt is a potassium salt of an organic acid.

4. A process according to any one of claims 1 to 3 **characterised by** concentrating the solution of potassium clavulanate in the aqueous medium.

5. A process according to any one of claims 1 to 4 **characterised in that** the precipitating solvent is a water miscible solvent optionally admixed with a water immiscible solvent.

6. A process according to any one of claims 1 to 5 **characterised in that** the titre of clavulanic acid is in the range 100,000 to 200,000 micrograms per ml.

7. A process according to any one of claims 1 to 6 **characterised in that** the clavulanic acid is obtained by a whole broth fermentation.

8. A process according to claim 7 **characterised in that** the whole broth fermentation is filtered.

9. A process according to claim 8 **characterised in that** the filtration is by ultrafiltration.

10. A process according to claim 9 **characterised in that** the filtration is followed by reverse osmosis.

11. A process according to claim 9 **characterised in that** the filtration is by RVF (rotary vacuum filtration).

12. A process according to claim 11 **characterised in that** the filtration is followed by ion exchange chromatography.

## Claims (Claims for the following Contracting State(s): AT)

1. A process for the preparation of pharmaceutically acceptable quality potassium clavulanate by the direct precipitation of clavulanic acid as the potassium salt which has not been pre-purified by the formation of an intermediate amine salt, by the reaction of clavulanic acid in solution with potassium ions in solution, in which the free acid of clavulanic acid resulting from a fermentation process is extracted into an organic solvent to thereby form a solution of clavulanic acid in the organic solvent, ***characterised* by**:
adding a potassium salt to the clavulanic acid in solution in the organic solvent,
then back extracting the so-formed potassium clavulanate into an aqueous medium from the organic solvent to thereby form a solution of potassium clavulanate in the aqueous medium as a separate phase to the organic solvent,
then causing the potassium clavulanate product to precipitate from the aqueous solution by the addition of an organic precipitating solvent to the aqueous solution of potassium clavulanate,
with the proviso that:
a process for the preparation of potassium clavulanate, wherein clavulanic acid in solution in a wholly or partly water-imiscible organic solvent is contacted in a contact region which is a region of high turbulence and/or shear stress, with a compound of potassium with a counter anion in solution or suspension, the counter anion being capable of exchange with clavulanate anion, in the presence of water, such that a solution of potassium clavulanate in an aqueous phase is formed, then the organic solvent and aqueous phases are physically separated during a separation step, followed by a further processing step in which the said salt of clavulanic acid is isolated from solution as a solid; and
a process for the preparation of clavulanic acid wherein (A) a coagulation step wherein a flocculating agent selected from calcium chloride, potassium ferrocyanide, water-soluble cationic polymers and organic polyelectrolyte is added to a fermentation broth of *Streptomyces Clavuligerus* at a temperature of from 0°C to + 10°C and at a pH of 4.0 - 4.5, (B) ultrafiltration of the clarified fermentation broth of Streptomyces Clavuligerus from the above step held at a temperature between 0°C to + 10°C and at a pH of between 6.0 and 6.5 through an ultrafiltration membrane that has a cut off between 30, 000 and 100, 000 Dalton, (C) decolourising the ultrafiltrate from the above step by running the same through a column containing a polymerised acrylic ester at a temperature between 0°C to + 10°C, (D) concentrating the liquid phase from (C) with the help of reverse osmosis through a membrane-which has a cut off from 750 to 500 Dalton, at a temperature between 0°C to + 10°C and at a pH of between 6.0 and 6.5, (E) decolourising the concentrated liquid phase with acid-washed activated carbon at a pH of between 6.0 and 6.5 and a temperature between 0°C to + 10°C, (F) salt-forming of the clavulanic acid through the addition of an aqueous solution of a potassium salt to the above obtained enriched aqueous solution, (G) precipitating the poassium salt of the clavulanic acid from the above-obtained aqueous solution through addition of a lower alcohol selected from methanol and ethanol and followed by addition of a solvent selected from isopropanol and acetone to the aqueous/alcoholic phase at a temperature between +5°C to + 15°C, is excluded.

2. A process according to claim 1 **characterised in that** the pharmaceutically acceptable quality potassium clavulanate has a purity of at least 80% as the pure free acid.

3. A process according to claim 1 or 2 **characterised in that** the potassium salt is a potassium salt of an organic acid.

4. A process according to any one of claims 1 to 3 **characterised by** concentrating the solution of potassium clavulanate in the aqueous medium.

5. A process according to any one of claims 1 to 4 **characterised in that** the precipitating solvent is a water miscible solvent optionally admixed with a water immiscible solvent.

6. A process according to any one of claims 1 to 5 **characterised in that** the titre of clavulanic acid is in the range 100,000 to 200,000 micrograms per ml.

7. A process according to any one of claims 1 to 6 **characterised in that** the clavulanic acid is obtained by a whole broth fermentation.

8. A process according to claim 7 **characterised in that** the whole broth fermentation is filtered.

9. A process according to claim 8 **characterised in that** the filtration is by ultrafiltration.

10. A process according to claim 9 **characterised in that** the filtration is followed by reverse osmosis.

11. A process according to claim 9 **characterised in that** the filtration is by RVF (rotary vacuum filtration).

12. A process according to claim 11 **characterised in that** the filtration is followed by ion exchange chromatography.

## Patentansprüche (Patentansprüche für folgende(n) Vertragsstaat(en): BE, CH, LI, DE, DK, ES, FI, FR, GB, GR, IE, IT, LU, MC, NL, PT, SE)

1. Verfahren zur Herstellung von Kaliumclavulanat pharmazeutisch akzeptabler Qualität durch das direkte Ausfällen von Clavulansäure als Kaliumsalz, das nicht durch die Bildung eines intermediären Aminsalzes vorgereinigt worden ist, durch die Umsetzung von Clavulansäure in Lösung mit Kaliumionen in Lösung, bei dem die freie Säure von Clavulansäure, die aus einem Fermentationsprozess resultiert, in ein organisches Lösungsmittel extrahiert wird, um dadurch eine Lösung von Clavulansäure in dem organischen Lösungsmittel zu bilden, **gekennzeichnet durch**:
Zusetzen eines Kaliumsalzes zur Clavulansäure in Lösung in dem organischen Lösungsmittel,
dann Rückextrahieren des so gebildeten Kaliumclavulanats aus dem organischen Lösungsmittel in ein wässriges Medium, um dadurch eine Lösung von Kaliumclavulanat in dern wässrigen Medium als getrennte Phase zu dem organischen Lösungsmittel zu bilden,
dann zu bewirken, dass das Kaliumclavulanatprodukt durch die Zugabe eines organischen Fällungslösungsmittels zu der wässrigen Lösung von Kaliumclavulanat aus der wässrigen Lösung ausfällt, mit der Maßgabe, dass:
ein Verfahren zur Herstellung von Kaliumclavulanat ausgeschlossen ist, bei dem Clavulansäure in Lösung in einem völlig oder teilweise mit Wasser nicht mischbaren organischen Lösungsmittel in einem Kontaktbereich, der ein Bereich hoher Durchwirbelung und/oder Scherbeanspruchung ist, mit einer Kaliumverbindung mit einem Gegenanion in Lösung oder Suspension, wobei das Gegenanion mit dem Clavulanatanion austauschen kann, in *Gegenwart* von Wasser so zusammengebracht wird, dass eine Lösung von Kaliumclavulanat in einer wässrigen Phase gebildet wird, dann das organische Lösungsmittel und die wässrigen Phasen während eines Trennungsschrittes physikalisch getrennt werden, worauf ein weiterer Verfahrensschritt folgt, bei dem das genannte Salz der Clavulansäure aus der Lösung als Feststoff isoliert wird.

2. Verfahren nach Anspruch 1, **dadurch gekennzeichnet, dass** das Kaliumclavulanat pharmazeutisch akzeptabler Qualität eine Reinheit von mindestens 80% als reine freie Säure aufweist.

3. Verfahren nach Anspruch 1 oder 2, **dadurch gekennzeichnet, dass** das Kaliumsalz ein Kaliumsalz einer organischen Säure ist.

4. Verfahren nach einem der Ansprüche 1 bis 3, **dadurch gekennzeichnet, dass** die Lösung von Kaliumclavulanat in dem wässrigen Medium eingeengt wird.

5. Verfahren nach einem der Ansprüche 1 bis 4, **dadurch gekennzeichnet, dass** das Fällungslösungsmittel ein mit Wasser mischbares Lösungsmittel ist, das gegebenenfalls mit einem mit Wasser nicht mischbaren Lösungsmitiel gemischt ist.

6. Verfahren nach einem der Ansprüche 1 bis 5, **dadurch gekennzeichnet, dass** der Titer der Clavulansäure im Bereich von 100,000 bis 200,000 Mikrogramm pro ml liegt.

7. Verfahren nach einem der Ansprüche 1 bis 6, **dadurch gekennzeichnet, dass** die Clavulansäure durch eine vollständige Nährlösungsfermentation erhalten wird.

8. Verfahren nach Anspruch 7, **dadurch gekennzeichnet, dass** die vollständige Nährlösungsfermentation filtriert wird.

9. Verfahren nach Anspruch 8, **dadurch gekennzeichnet, dass** die Filtration durch Ultrafiltration erfolgt.

10. Verfahren nach Anspruch 9, **dadurch gekennzeichnet, dass** der Filtration eine Umkehrosmose folgt.

11. Verfahren nach Anspruch 9, **dadurch gekennzeichnet, dass** die Filtration durch RVF (Rotationsvakuumfiltration) erfolgt.

12. Verfahren nach Anspruch 11, **dadurch gekennzeichnet, dass** der Filtration eine Ionenaustauschchromatographie folgt.

## Patentansprüche (Patentansprüche für folgende(n) Vertragsstaat(en): AT)

1. Verfahren zur Herstellung von Kaliumclavulanat pharmazeutisch akzeptabler Qualität durch das direkte Ausfällen von Clavulansäure als Kaliumsalz, das nicht durch die Bildung eines intermediären Aminsalzes vorgereinigt worden ist, durch die Umsetzung von Clavulansäure in Lösung mit Kaliumionen in Lösung, bei dem die freie Säure von Clavulansäure, die aus einem Fermentationsprozess resultiert, in ein organisches Lösungsmittel extrahiert wird, um dadurch eine Lösung von Clavulansäure in dem organischen Lösungsmittel zu bilden, **gekennzeichnet durch**:
Zusetzen eines Kaliumsalzes zur Clavulansäure in Lösung in dem organischen Lösungsmittel,
dann Rückextrahieren des so gebildeten Kaliumclavulanats aus dem organischen Lösungsmittel in ein wässriges Medium, um dadurch eine Lösung von Kaliumclavulanat in dem wässrigen Medium als getrennte Phase zu dem organischen Lösungsmittel zu bilden,
dann zu bewirken, dass das Kaliumclavulanatprodukt durch die Zugabe eines organischen Fällungslösungsmittels zu der wässrigen Lösung von Kaliumclavulanat aus der wässrigen Lösung ausfällt,
mit der Maßgabe, dass ausgeschlossen sind:
ein Verfahren zur Herstellung von Kaliumclavulanat, bei dem Clavulansäure in Lösung in einem völlig oder teilweise mit Wasser nicht mischbaren organischen Lösungsmittel in einem Kontaktbereich, der ein Bereich hoher Durchwirbelung und/oder Scherbeanspruchung ist, mit einer Kaliumverbindung mit einem Gegenanion in Lösung oder Suspension, wobei das Gegenanion mit dem Clavulanatanion austauschen kann, in Gegenwart von Wasser so zusammengebracht wird, dass eine Lösung von Kaliumclavulanat in einer wässrigen Phase gebildet wird, dann das organische Lösungsmittel und die wässrigen Phasen während eines Trennungsschrittes physikalisch getrennt werden, worauf ein weiterer Verfahrensschritt folgt, bei dem das genannte Salz der Clavulansäure aus der Lösung als Feststoff isoliert wird; und
ein Verfahren zur Herstellung von Clavulansäure, bei dem (A) ein Koagulationsschritt, bei dem ein Flockungsmittel, das aus Calciumchlorid, Kaliumhexacyanoferrat(II), wasserlöslichen kationischen Polymeren und organischem Polyelektrolyt ausgewählt ist, einer Fermentationsbrühe von *Streptomyces Clavuligerus* bei einer Temperatur von 0°C bis +10°C und bei einem pH-Wert von 4,0 - 4,5 zugesetzt wird, (B) Ultrafiltration der geklärten Fermentationsbrühe von *Streptomyces Clavuligerus* aus dem vorstehenden Schritt, die bei einer Temperatur zwischen 0°C bis +10°C und bei einem pH-Wert zwischen 6,0 und 6,5 gehalten wird, durch eine Ultrafiltrationsmembran, die ein Rückhaltevermögen zwischen 30,000 und 100,000 Dalton aufweist, (C) Entfärben des Ultrafiltrats aus dem vorstehenden Schritt durch Laufen desselben durch eine Säule, die einen polymerisierten Acrylester enthält, bei einer Temperatur zwischen 0°C bis + 10°C, (D) Einengen der flüssigen Phase aus (C) mit Hilfe einer Umkehrosmose durch eine Membran, die ein Rückhaltevermögen von 750 bis 500 Dalton aufweist, bei einer Temperatur zwischen 0°C bis +10°C und bei einem pH-Wert zwischen 6,0 und 6,5, (E) Entfärben der eingeengten flüssigen Phase mit säuregewaschener Aktivkohle bei einem pH-Wert zwischen 6,0 und 6,5 und einer Temperatur zwischen 0°C bis + 10°C, (F) Salzbildung aus der Clavulansäure durch den Zusatz einer wässrigen Lösung eines Kaliumsalzes zu der vorstehend erhaltenen angereicherten wässrigen Lösung, (G) Ausfällen des Kaliumsalzes der Clavulansäure aus der vorstehend erhaltenen wässrigen Lösung durch Zusatz eines niederen Alkohols, der aus Methanol und Ethanol ausgewählt ist, und gefolgt von einem Zusatz eines Lösungsmittels, das aus Isopropanol und Aceton ausgewählt ist, zu der wässrigen/alkoholischen Phase bei einer Temperatur zwischen +5°C bis +15°C.

2. Verfahren nach Anspruch 1, **dadurch gekennzeichnet, dass** das Kaliumclavulanat pharmazeutisch akzeptabler Qualität eine Reinheit von mindestens 80% als reine freie Säure aufweist.

3. Verfahren nach Anspruch 1 oder 2, **dadurch gekennzeichnet, dass** das Kaliumsalz ein Kaliumsalz einer organischen Säure ist.

4. Verfahren nach einem der Ansprüche 1 bis 3, **dadurch gekennzeichnet, dass** die Lösung von Kaliumclavulanat in dem wässrigen Medium eingeengt wird.

5. Verfahren nach einem der Ansprüche 1 bis 4, **dadurch gekennzeichnet, dass** das Fällungslösungsmittel ein mit Wasser mischbares Lösungsmittel ist, das gegebenenfalls mit einem mit Wasser nicht mischbaren Lösungsmittel gemischt ist.

6. Verfahren nach einem der Ansprüche 1 bis 5, **dadurch gekennzeichnet, dass** der Titer der Clavulansäure im Bereich von 100,000 bis 200,000 Mikrogramm pro ml liegt.

7. Verfahren nach einem der Ansprüche 1 bis 6, **dadurch gekennzeichnet, dass** die Clavulansäure durch eine vollständige Nährlösungsfermentation erhalten wird.

8. Verfahren nach Anspruch 7, **dadurch gekennzeichnet, dass** die vollständige Nährlösungsfermentation filtriert wird.

9. Verfahren nach Anspruch 8, **dadurch gekennzeichnet, dass** die Filtration durch Ultrafiltration erfolgt.

10. Verfahren nach Anspruch 9, **dadurch gekennzeichnet, dass** der Filtration eine Umkehrosmose folgt.

11. Verfahren nach Anspruch 9, **dadurch gekennzeichnet, dass** die Filtration durch RVF (Rotationsvakuumfiltration) erfolgt.

12. Verfahren nach Anspruch 11, **dadurch gekennzeichnet, dass** der Filtration eine Ionenaustauschchromatographie folgt.

## Revendications (Revendications pour l'(les) Etat(s) contractant(s) suivant(s): BE, CH, LI, DE, DK BE, CH, LI, DE, DK, ES, FI, FR, GB, GR, IE, IT, LU, MC, NL, PT, SE)

1. Procédé pour la préparation de clavulanate de potassium de qualité pharmaceutiquement acceptable par la précipitation directe d'acide clavulanique sous forme du sel de potassium qui n'a pas été prépurifié par formation d'un sel d'amine intermédiaire, par la réaction de l'acide clavulanique en solution avec des ions potassium en solution, dans lequel l'acide libre d'acide clavulanique résultant d'un procédé de fermentation est extrait dans un solvant organique pour former ainsi une solution d'acide clavulanique dans le solvant organique, **caractérisé par** :
l'addition d'un sel de potassium à l'acide clavulanique en solution dans le solvant organique,
puis la réextraction du clavulanate de potassium ainsi formé dans un milieu aqueux à partir du solvant organique pour former ainsi une solution de clavulanate de potassium dans le milieu aqueux sous forme d'une phase distincte de celle du solvant organique,
puis l'étape consistant à provoquer la précipitation du produit consistant en clavulanate de potassium à partir de la solution aqueuse par addition d'un solvant organique précipitant à la solution aqueuse de clavulanate de potassium,
sous réserve :
qu'un procédé pour la préparation de clavulanate de potassium, dans lequel de l'acide clavulanique en solution dans un solvant organique totalement ou partiellement non miscible à l'eau est mis en contact, dans une région de contact qui est une région de forte turbulence et/ou de forte tension de cisaillement, avec un composé de potassium ayant un anion complémentaire en solution ou en suspension, l'anion complémentaire étant apte à l'échange avec l'anion clavulanate, en présence d'eau, de telle sorte qu'une solution de clavulanate de potassium dans une phase aqueuse soit formée, puis la phase de solvant organique et la phase aqueuse sont physiquement séparées au cours d'une étape de séparation, avec ensuite une étape de traitement supplémentaire dans laquelle ledit sel d'acide clavulanique est isolé de la solution sous forme d'une substance solide, soit exclu.

2. Procédé suivant la revendication 1, **caractérisé en ce que** le clavulanate de potassium de qualité pharmaceutiquement acceptable a une pureté d'au moins 80 % sous forme de l'acide libre pur.

3. Procédé suivant la revendication 1 ou 2, **caractérisé en ce que** le sel de potassium est un sel de potassium d'un acide organique.

4. Procédé suivant l'une quelconque des revendications 1 à 3, **caractérisé par** la concentration de la solution de clavulanate de potassium dans le milieu aqueux.

5. Procédé suivant l'une quelconque des revendications 1 à 4, **caractérisé en ce que** le solvant précipitant est un solvant miscible à l'eau, facultativement en mélange avec un solvant non miscible à l'eau.

6. Procédé suivant l'une quelconque des revendications 1 à 5, **caractérisé en ce que** le titre d'acide clavulanique est compris dans l'intervalle de 100 000 à 200 000 microgrammes par ml.

7. Procédé suivant l'une quelconque des revendications 1 à 6, **caractérisé en ce que** l'acide clavulanique est obtenu par une fermentation de bouillon entier.

8. Procédé suivant la revendication 7, **caractérisé en ce que** le produit de la fermentation de bouillon entier est filtré.

9. Procédé suivant la revendication 8, **caractérisé en ce que** la filtration est effectuée par ultrafiltration.

10. Procédé suivant la revendication 9, **caractérisé en ce que** la filtration est suivie par une osmose inverse.

11. Procédé suivant la revendication 9, **caractérisé en ce que** la filtration est effectuée par RVF (filtration rotative sous vide).

12. Procédé suivant la revendication 11, **caractérisé en ce que** la filtration est suivie par une chromatographie d'échange d'ions.

## Revendications (Revendications pour l'(les) Etat(s) contractant(s) suivant(s): AT)

1. Procédé pour la préparation de clavulanate de potassium de qualité pharmaceutiquement acceptable par la précipitation directe d'acide clavulanique sous forme du sel de potassium qui n'a pas été prépurifié par formation d'un sel d'amine intermédiaire, par la réaction d'acide clavulanique en solution avec des ions potassium en solution, dans lequel l'acide libre d'acide clavulanique résultant d'un procédé de fermentation est extrait dans un solvant organique pour former ainsi une solution d'acide clavulanique dans le solvant organique, **caractérisé par** :
l'addition d'un sel de potassium à l'acide clavulanique en solution dans le solvant organique,
puis la réextraction du clavulanate de potassium ainsi formé dans un milieu aqueux à partir du solvant organique pour former ainsi une solution de clavulanate de potassium dans le milieu aqueux sous forme d'une phase distincte de celle du solvant organique,
puis l'étape consistant à provoquer la précipitation du produit consistant en clavulanate de potassium à partir de la solution aqueuse par addition d'un solvant organique précipitant à la solution aqueuse de clavulanate de potassium,
sous réserve :
qu'un procédé pour la préparation de clavulanate de potassium, dans lequel l'acide clavulanique en solution dans un solvant organique totalement ou partiellement non miscible à l'eau est mis en contact, dans une région de contact qui est une région de forte turbulence et/ou de forte tension de cisaillement, avec un composé de potassium avec un anion complémentaire en solution ou suspension, l'anion complémentaire étant apte à l'échange avec l'anion clavulanate, en présence d'eau, de telle sorte qu'une solution de clavulanate de potassium dans une phase aqueuse soit formée, puis la phase de solvant organique et la phase aqueuse sont physiquement séparées au cours d'une étape de séparation, avec ensuite une étape de traitement supplémentaire dans laquelle ledit sel d'acide clavulanique est isolé de la solution sous forme d'une substance solide; et
qu'un procédé pour la préparation d'acide clavulanique comprenant (A) une étape de coagulation dans laquelle un agent flocculent choisi entre le chlorure de calcium, le ferrocyanure de potassium, des polymères cationiques hydrosolubles et un polyélectrolyte organique, est ajouté à un bouillon de fermentation de Streptomyces clavuligerus à une température comprise dans l'intervalle de 0°C à +10°C et à un pH compris dans la plage de 4,0 à 4,5, (B) une ultrafiltration du bouillon de fermentation clarifié de Streptomyces clavuligerus de l'étape ci-dessus maintenu à une température comprise dans l'intervalle de 0°C à +10°C à un pH compris dans la plage de 6,0 à 6,5 à travers une membrane d'ultrafiltration qui a une valeur d'exclusion comprise dans l'intervalle de 30 000 à 100 000 daltons, (C) une décoloration de l'ultrafiltrat de l'étape ci-dessus en passant cet ultrafiltrat à travers une colonne contenant un ester acrylique polymérisé à une température comprise dans l'intervalle de 0°C à +10°C, (D) une concentration de la phase liquide de (C) par osmose inverse à travers une membrane qui a une valeur d'exclusion de 750 à 500 daltons, à une température comprise dans l'intervalle de 0°C à +10°C et un pH compris dans la plage de 6,0 à 6,5, (E) une décoloration de la phase liquide concentrée avec du charbon actif, lavé à l'acide à un pH compris dans la plage de 6,0 à 6,5 et à une température comprise dans l'intervalle de 0°C à +10°C, (F) la formation d'un sel de l'acide clavulanique par addition d'une solution d'un sel de potassium à la solution aqueuse enrichie obtenue ci-dessus, (G) une précipitation du sel de potassium d'acide clavulanique à partir de la solution aqueuse obtenue ci-dessus par addition d'un alcool inférieur choisi entre le méthanol et l'éthanol et ensuite par addition d'un solvant choisi entre l'isopropanol et l'acétone à la phase aqueuse/alcoolique à une température comprise dans l'intervalle de +5°C à +15°C, soient exclus.

2. Procédé suivant la revendication 1, **caractérisé en ce que** le clavulanate de potassium de qualité pharmaceutiquement acceptable a une pureté d'au moins 80 % sous forme de l'acide libre pur.

3. Procédé suivant la revendication 1 ou 2, **caractérisé en ce que** le sel de potassium est un sel de potassium d'un acide organique.

4. Procédé suivant l'une quelconque des revendications 1 à 3, **caractérisé par** la concentration de la solution de clavulanate de potassium dans le milieu aqueux.

5. Procédé suivant l'une quelconque des revendications 1 à 4, **caractérisé en ce que** le solvant précipitant est un solvant miscible à l'eau, facultativement en mélange avec un solvant non miscible à l'eau.

6. Procédé suivant l'une quelconque des revendications 1 à 5, **caractérisé en ce que** le titre d'acide clavulanique est compris dans l'intervalle de 100 000 à 200 000 microgrammes par ml.

7. Procédé suivant l'une quelconque des revendications 1 à 6, **caractérisé en ce que** l'acide clavulanique est obtenu par une fermentation de bouillon entier.

8. Procédé suivant la revendication 7, **caractérisé en ce que** le produit de la fermentation de bouillon entier est filtré.

9. Procédé suivant la revendication 8, **caractérisé en ce que** la filtration est effectuée par ultrafiltration.

10. Procédé suivant la revendication 9, **caractérisé en ce que** la filtration est suivie par une osmose inverse.

11. Procédé suivant la revendication 9, **caractérisé en ce que** la filtration est effectuée par RVF (filtration rotative sous vide).

12. Procédé suivant la revendication 11, **caractérisé en ce que** la filtration est suivie par une chromatographie d'échange d'ions.
